# EUROPÄISCHE PATENTANMELDUNG

(11) **EP 0 816 512 A1**
(43) Veröffentlichungstag der Anmeldung: **07.01.1998**
(21) Anmeldenummer: 97110315.5
(22) Anmeldetag: 24.06.1997
(51) Int. Cl.: C12Q 1/04, C12Q 1/66

(54) **Verwendung eines Biolumineszenz-Tests zum Nachweis von Mikroorganismen in Dispersionen, die Polymere und/oder Pigmente enthalten**

(30) Priorität: 24.06.1996 DE 19625137
(71) Anmelder: BASF AKTIENGESELLSCHAFT, 67056 Ludwigshafen (DE)
(72) Erfinder: Balk, Roelof, Dr., 67459 Böhl-Iggelheim (DE); Eipel, Heinz, 64625 Bensheim (DE); Pressler, Uwe, Dr., 67165 Waldsee (DE)
(74) Vertreter: Kinzebach, Werner, Dr.

(57) **Zusammenfassung**

Die Erfindung betrifft die Verwendung eines Biolumineszenz-Tests zum Nachweis von Mikroorganismen in Dispersionen, die Polymere und/oder Pigmente enthalten.

## Beschreibung

Die Erfindung betrifft die Verwendung eines Biolumineszenz-Tests zum Nachweis von Mikroorganismen in Dispersionen, die Polymere und/oder Pigmente enthalten.

Biolumineszenz-Reaktionen, insbesondere ATP-getriebene Lumineszenz-Reaktionen, wie die Luziferin/Luziferase-Reaktionen, sind bekannt, z.B. aus Römpp, Chemie-Lexikon, 9. Aufl., S. 425 bzw. S. 2552 f. Als Luziferasen bezeichnet man verschiedene strukturell unterschiedliche, zu den Oxidoreductasen gehörende Enzyme aus verschiedenen Organismen mit der gemeinsamen Eigenschaft, durch Oxidation von Luziferinen Biolumineszenz induzieren zu können. Luziferine sind strukturell völlig unterschiedliche Naturstoffe, die bei Einwirkung einer Luziferase Bioluminesienz erzeugen.

Am besten untersucht ist die Luziferin/Luziferase-Reaktion beim amerikanischen Leuchtkäfer Photinus pyralis, bei dem eine 550 Aminosäurereste umfassende Luziferase folgende Reaktion katalysiert: 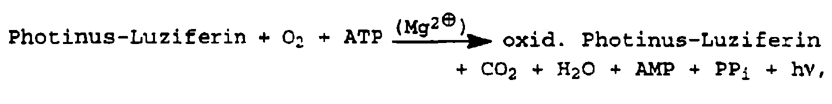 wobei folgende Definitionen gelten: ATP = Adenosin-5'-triphosphat, AMP = Adenosin-5'-monophosphat, PPᵢ = anorg. Diphosphat, hν = Licht, Photinus-Luziferin = (*S*)-4,5-Dihydro-2-(6-hydroxybenzothiazol-2-yl)-thiazol-4-carbonsäure.

Der erste Reaktionsschritt ist eine Diphosphat-Abspaltung und Bildung von Luciferyl-Adenylat (Anhydrid zwischen Luziferin und AMP). Die Reaktion des inzwischen geklonten Enzyms läßt sich unter anderem zum schnellen und empfindlichen Nachweis von ATP (bis zu 10⁻¹¹ molaren Konz.) verwerten.

Da ATP der bedeutendste biologische Energieträger ist, kann die Luziferin/Luziferase-Reaktion auch zum Nachweis von Mikroorganismen verwendet werden, wenn diese zuvor lysiert worden sind. Unter "lysieren" wird im Rahmen der vorliegenden Erfindung verstanden, Zellen so zu beschädigen, daß sie ATP freisetzen.

Aus der WO 96/07759 ist bekannt, daß die Lyse von Mikrobenzellen durch Behandlung mit kochendem Puffer, mit Lösungsmitteln, mit Säuren oder mit Tensiden erfolgen kann. Hinsichtlich der Lyse mit Tensiden wird darauf hingewiesen, daß ein Gemisch aus nichtionischen und kationischen Tensiden unwirksam ist, weil nichtionische Tenside Bakterien vor der Lyse durch kationische Tenside bewahren (S. 1, Z. 25-28).

Des weiteren wird in der WO 96/07759 ein Testkit beschrieben, der Luziferin, Luziferase, einen Puffer sowie ein tensidisches Lysemittel zusammen mit einer mittelkettigen Fettsäure enthält. Die Fettsäure hat den Zweck, die Luziferase vor Inaktivierung durch das tensidische Lysemittel zu schützen. Der Testkit ist zur Bestimmung von aus Mikroorganismen freigesetztem ATP geeignet. Spezielle Einsatzgebiete für diesen Testkit sind darin nicht genannt.

Ein entsprechender Testkit wird von der Anmelderin der WO 96/07759 (Fa. Celsis (GB)) in verschiedenen Variationen (z.B. "Hygiene Monitoring Kit", "Low Decay Rate Bioluminescence Kit") jedoch stets mit den gleichen Tensiden als Bestandteilen des lysierenden Mittels, vertrieben. Den Produktspezifikationen, beispielsweise für das "Low Decay Rate Bioluminescence Kit", ist zu entnehmen, daß die Anwesenheit von Tensiden in den zu untersuchenden Proben den Test stören kann. Als Anwendungsgebiete werden in den Produktspezifikationen ausschließlich die Qualitätskontrolle von Lebensmitteln, Wasser, Getränken, Kosmetika, Körperpflegemitteln und die Hygieneüberwachung von kosmetischen, pharmazeutischen und biotechnologischen Fertigungsanlagen genannt.

Die Verwendung von Biolumineszenz-Tests auf der Basis von tensidischer Lyse zum Nachweis von Mikroorganismen in Nahrungsmitteln und in Körperflüssigkeiten, wie Milch, Urin, Blut, der zentralen Rückenmarksflüssigkeit oder Speichel, ist auch aus der DE-B-2823916 bekannt.

Es liegt die Ankündigung eines am 25./26. Juni 1996 im Rahmen des "International Symposium on Industrial Applications of Bioluminescence in Microbiology" in England von Dr. Michael Cresswell (National Starch & Chemical Co. Europe) zu haltenden Vortrages mit dem Titel: "The application of ATP technology in the adhesives and polymer dispersion industries" vor. Wie, zu welchem Zweck und mit welchen Methoden die Anwendung der ATP-Technologie erfolgen soll, ist aber nicht bekannt.

Es mußte also davon ausgegangen worden, daß auf tensidischer Lyse basierende Biolumineszenz-Tests nicht zum Nachweis von Mikroorganismen in Dispersionen, die Polymere und/oder Pigmente enthalten, verwendet werden können, insbesondere nicht in Dispersionen, die anionische oder nichtionische Tenside enthalten. Eigene Untersuchungen bestätigen dieses Vorurteil insofern, als eine unmittelbare Anwendung bei Dispersionen mit eine Feststoffgehalt von 10 bis 80 Gew.-% und einem Gehalt an meist anionischen und/oder nichtionischen Emulgatoren von 0,1 bis 10 Gew.-%, bezogen auf den Feststoffgehalt, nicht möglich war.

So wird der Nachweis von Mikroorganismen in Dispersionen, die Polymere und/oder Pigmente enthalten, herkömmlicherweise durch Wachstumstests wie z.B. Easycult-TTC (erhältlich von Orion Diagnostica, Finnland) oder Agar-Plattierung geführt. Diese Methoden funktionieren jedoch nur indirekt nach Zugabe eines Nährmediums und 24 bis 48 stündiger Inkubation bei erhöhter Temperatur (etwa 35 bis 37°C). Besonders nachteilig ist hierbei die lange Wartezeit zwischen Probenentnahme und Untersuchungsergebnis. Dies führt häufig zur prophylaktischen Anwendung desinfizierender Mittel, die teuer und toxisch nicht völlig unbedenklich sind, wenn sie z.B. in Dispersionen für Anstrichfarben verwendet werden. Außerdem können Konservierungsmittel in höheren Konzentrationen die Materialeigenschaften der Dispersionen beeinträchtigen.

Mikroorganismenbefall kann aber nicht einfach hingenommen werden, da er, neben der Geruchsbelästigung, vor allem gravierende technische Nachteile mit sich bringt. Die befallenen Dispersionen können ein verändertes Fließverhalten zeigen, was sie für die Verwendung in industriellen Lackier- oder Beschichtungsanlagen, die auf ein bestimmtes Fließverhalten der Dispersionen eingestellt sind, untauglich machen kann. Wird Druckpapier mit befallenen Dispersionen beschichtet, kann es aufgrund der verschlechterten Materialeigenschaften der Dispersion auf den schnellen Rotationsmaschinen der Druckereien reißen. Mit befallenen Dispersionen erzeugte Filme können verspröden oder sich verfärben. Da die Mikroorganismen in den Dispersionen Gasblasen bilden, kann es zu Kratern, beispielsweise in Lackfilmen auf Kraftfahrzeugkarosserien kommen. Organische Säuren, die von den Mikroorganismen gebildet werden und somit in den Dispersionen, die Polymere und/oder Pigmente enthalten, vorhanden sind, können Lagertanks, Fässer oder sonstige Metallgegenstände korrodieren,

Es ist demzufolge wünschenswert, mikrobielle Kontaminationen von Dispersionen, die Polymere und/oder Pigmente enthalten, zuverlässig und schnell erkennen und dadurch den Einsatz von Desinfektionsmitteln auf ein Minimum beschränken zu können.

Überraschenderweise wurde nunmehr gefunden, daß mittels eines auf tensidischer Lyse basierenden Biolumineszenz-Tests mikrobielle Kontaminationen auch in tensidhaltigen Dispersionen, die Polymere und/oder Pigmente enthalten, zuverlässig und schnell erkannt werden können.

Gegenstand der Erfindung ist somit die Verwendung eines Biolumineszenz-Tests zum Nachweis von Mikroorganismen in Dispersionen, die Polymere und/oder Pigmente enthalten.

Der Biolumineszenz-Test ist erfindungsgemäß für die Untersuchung aller Arten von wäßrigen Dispersionen, die Polymere und/oder Pigmente enthalten, geeignet. Bevorzugt brauchbar ist er jedoch zur Untersuchung von Dispersionen auf Vinylchlorid-, Butadien- und Styrolbasis und auf Basis von Estern einer α,β-ethylenisch ungesättigten C₃-C₆-Mono- oder Dicarbonsäure, wie Acrylsäure, Methacrylsäure, Maleinsäure, Fumarsäure oder Itaconsäure, mit C₁-C₃₀-Alkanolen, insbesondere C₁-C₁₂-Alkanolen, wie Methanol, Ethanol, n-Propanol, i-Propanol, n-Butanol, iso-Butanol, sek.-Butanol, tert.-Butanol, 2-Ethylhexyl, Decanol, Dodecanol etc. Bevorzugte Acrylate sind solche auf Basis von (Meth)acrylsäure mit C₁-C₄-Alkanolen, insbesondere C₄-Alkanolen.

Es kann sich bei den Dispersionen, die Polymere und/oder Pigmente enthalten, um Homopolymere oder Copolymere handeln. Die Copolymere auf Basis der erwähnten Ester können mindestens ein weiteres Monomer, wie (Meth)acrylsäure, (Meth)acrylamid, N-C₁-C₆-Alkyl- oder N-C₁-C₆-Hydroxyalkylsubstituierte (Meth) acrylamide, Hydroxyalkyl(methacrylate), z.B. Hydroxyethyl(meth)acrylat, Vinylester, z.B. Vinylacetat, Vinylpropionat, vinylaromatische Verbindungen, z.B. Styrol, α-Methylstyrol, und Olefine, z.B. Ethylen, Propylen oder Butadien, und (Meth)acrylnitril einpolymerisiert enthalten. Copolymere auf Styrolbasis können ebenfalls mindestens eines der genannten Monomere (die von Styrol verschieden sind) einpolymeriziert enthalten. Styrol-Butadien und Acrylat-Styrol-Dispersionen sind besonders bevorzugt.

Die Herstellung der brauchbaren Dispersionen, die Polymere und/oder Pigmente enthalten, kann in üblicher Weise durch radikalische Suspensions- oder Emulsionspolymerisation in Anwesenheit üblicher Initiatoren und Emulgatoren oder Schutzkolloide erfolgen, wie beispielsweise in Ullmanns Encyclopedia of Industrial Chemistry, 5th edition, vol. A21, S. 373-393 oder in Houben-Weyl, Band XIV/1, Makromolekulare Stoffe, Georg-Thieme-Verlag, Stuttgart 1961, S. 411-420 bzw. S. 192-208 beschrieben. Brauchbare Initiatoren sind insbesondere Peroxoverbindungen, wie Wasserstoffperoxid, Alkalimetallperoxodisulfate, Hydroperoxide, Dialkylperoxide, Dibenzoylperoxid etc. sowie Azoverbindungen und Redoxinitiatoren. Brauchbare Emulgatoren sind z.B. ethoxylierte Mono-, Di- und Trialkylphenole (EO-Grad: 3 bis 50, Alkylrest: C₄-C₉), ethoxylierte Fettalkohole (EO-Grad: 3 bis 50, Alkylrest: C₈-C₃₆) sowie insbesondere anionische Emulgatoren, wie Alkali- und Ammoniumsalze von Alkylsulfaten oder Alkylethersulfaten, von Alkylsulfonsäuren oder Alkyldiphenyloxidsulfonate. Weitere geeignete anionische Emulgatoren sind Bis(phenylsulfonsäure)ether bzw. deren Alkali- oder Anmoniumsalze, die an einem oder beiden aromatischen Ringen eine C₄-C₂₄-Alkylgruppe tragen. Geeignete Schutzkolloide sind insbesondere Polyvinylalkohol, Cellulosederivate oder Polyvinylpyrrolidon.

Zur Verringerung des Molekulargewichtes kann die Polymerisation in Anwesenheit üblicher Regler durchgeführt werden. Brauchbare Regler sind insbesondere Aldehyde oder organische Halogen- oder Schwefelverbindungen, wie Formaldehyd, Acetaldehyd, Bromtrichlormethan, Mercaptoethanol, 2-Ethylhexylthioglycolat, Thioglykolsäure oder Dodecylmercaptan.

Gegenstand der Erfindung ist auch ein Verfahren zum Nachweis von Mikroorganismen in Dispersionen, die Polymere und/oder Pigmente enthalten, wobei man
a) eine Probe der Dispersion mit Wasser verdünnt,
b) Mikroorganismen-lysierende Agenzien zu der verdünnten Probe hinzufügt und einwirken läßt,
c) eine Mischung aus Luziferin und Luziferase zugibt und
d) die Lumineszenz der Probe mißt.

Der zu untersuchenden Dispersion wird eine Probe entnommen, die so verdünnt wird, daß ein Gewichtsverhältnis von etwa 1 Teil Polymer auf etwa 10 bis 300 Teile Wasser, vorzugsweise von etwa 1 Teil Polymer auf etwa 100 bis 200 Teile Wasser, vorliegt. Etwa 300 bis 500 µl dieser Probe werden in ein geeignetes Gefäß, z.B. ein Meßröhrchen gegeben, das in ein empfindliches, mit Photomultipliern versehenes Luminometer gestellt wird. Dann gibt man Mikroorganismen-lysierende Agenzien zu und läßt diese mindestens 5 bis 20 sec., vorzugsweise mindestens 10 sec. einwirken. Als Mikroorganismen-lysierende Agenzien sind die als oberflächenaktive Mittel in der DE-B-2823916 und der WO-96/07759 beschriebenen Agenzien verwendbar. Besonders bevorzugt sind kationische oder in situ kationisch wirkende Tenside, beispielsweise ethoxylierte Amine, ethoxylierte Diamine, quaternäre Ammoniumsalze eines ethoxylierten Amins sowie Mischungen davon und Fettsäure-Polyethylenglykolester, die jeweils zusammen mit einer Fettsäure mittlerer Kettenlänge eingesetzt werden können. Ganz besonders bevorzugt sind kationische Tenside wie quaternäre Aumoniumsalze. Vorzugsweise beträgt die Tensidkonzentration etwa 0,01 bis 5 Gew.-%, bezogen auf den Gesamtonsatz. Anschließend werden etwa 50 bis 150 µl, vorzugsweise 80 bis 120 µl einer Mischung aus einem Luziferin und einer Luziferase zugegeben, wobei eine Mischung aus Photinus-Luziferin und Photinus-Luziferase besonders bevorzugt ist. Die Lumineszenz wird dann mindestens 10 bis 20 sec., insbesondere mindestens 15 sec. gemessen und wie in der Luminometrie üblich in "relative light units" (RLU) ausgedrückt.

Die folgenden Beispiele erläutern die Erfindung, ohne sie jedoch darauf einzuschränken.

### Beispiel 1

### Vergleich des Biolumineszenz-Schnelltests mit einem herkömmlichen mikrobiologischen Wachstumstest (Easycult-TTC)

Es wurden Proben verschiedener Polymerdispersionen gezogen, die mit Mikroorganismen kontaminiert waren. Verwendet wurden Dispersionen von Styrolpolymeren sowie Dispersionen von Acrylsäureester-Styrol-Copolymeren. Die genaue Zusammensetzung der Polymere, die in den Dispersionen enthaltenen Emulgatoren und Initiatoren sowie der pH-Bereich der Dispersionen sind der Tabelle 1 zu entnehmen. Für die Tests wurde ein von der Fa. Celsis als "Hygiene Monitoring Kit" vertriebener Satz an Reaktionsgefäßen und Reagenzien verwendet. Die Proben wurden mit vollentsalztem (Leitfähigkeit etwa 0,05 µS/cm) und über einen 0,2 µm Mikrofilter steril filtriertem Wasser im Verhältnis 1:100 verdünnt. Bei einem Feststoffgehalt der Dispersionen von etwa 30 bis 80 Gew.-% führte dies zu einem Gewichtsverhältnis von etwa 1 Teil Polymer auf etwa 125 bis 333 Teile Wasser. Jeweils 400 µl der verdünnten Proben wurden in ein Meßröhrchen pipettiert und in ein Luminometer des Typs Optocomp I (erhältlich von der Fa. Celsis) gestellt. Danach wurden 200 µl Lysisreagens, das als wirksame Bestandteile im wesentlichen kationische Tenside (0,2 Gew.-%), vor allem quaternäre Ammoniumsalze von ethoxylierten Aminen mit 9 bis 12 Kohlenstoffatomen in der Kette, und kleinere Mengen anionischer (0,05 Gew.-%) sowie nichtionischer (0,05 Gew.-%) Tenside enthielt, zugegeben. Nach einer Reaktionszeit von 10 sec. wurden 100 µl der Luziferin/Luziferase-Mischung zugegeben und die Lumineszenz nach einer Wartezeit von 0,5 sec. für eine Dauer von 15 sec. gemessen. Die Ergebnisse der Messungen sind in Tabelle 2 gezeigt. Zum Vergleich sind in Tabelle 2 auch die Ergebnisse des herkömmlichen Wachstumstests (Easycult-TTC) angegeben.

Eine sterile Dispersionsprobe (Nullprobc) ergab einen RLU-Wert von 2,3 x 10². Die in Tabelle 2 angegebenen RLU-Werte liegen deutlich höher und sind somit signifikant für den Nachweis von Mikroorganismen.

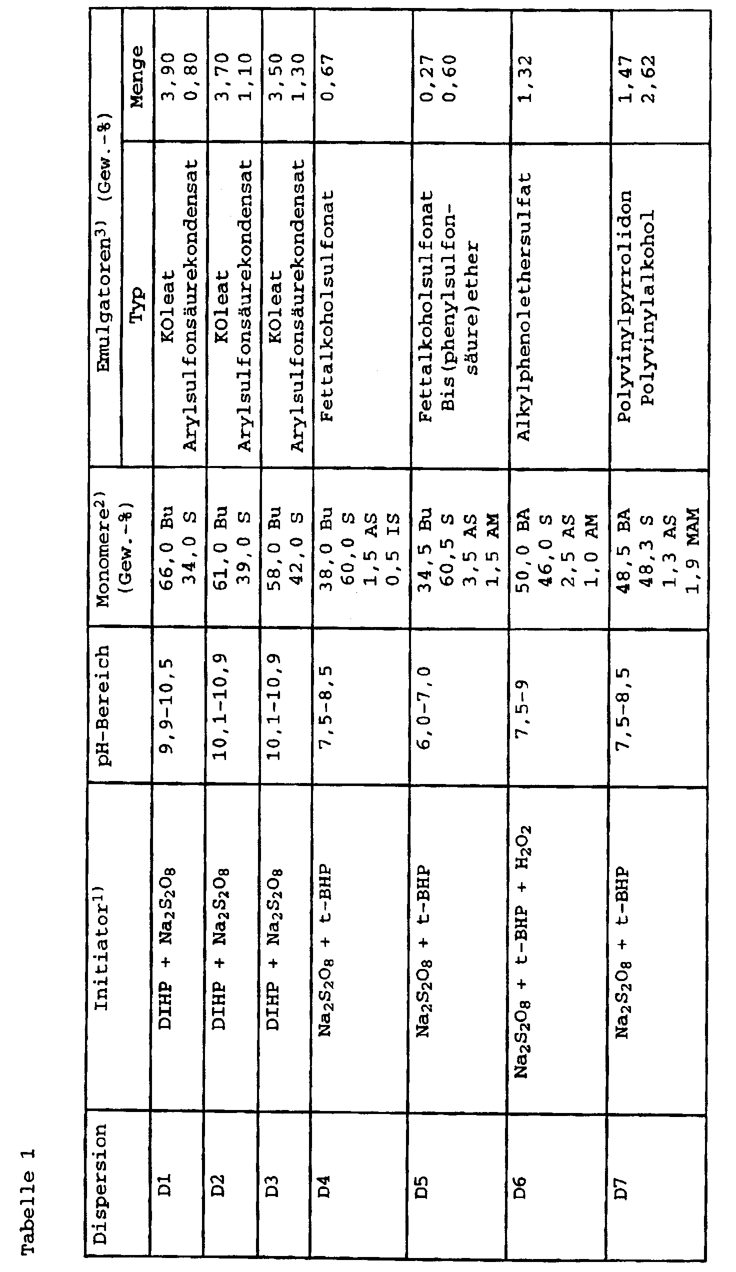

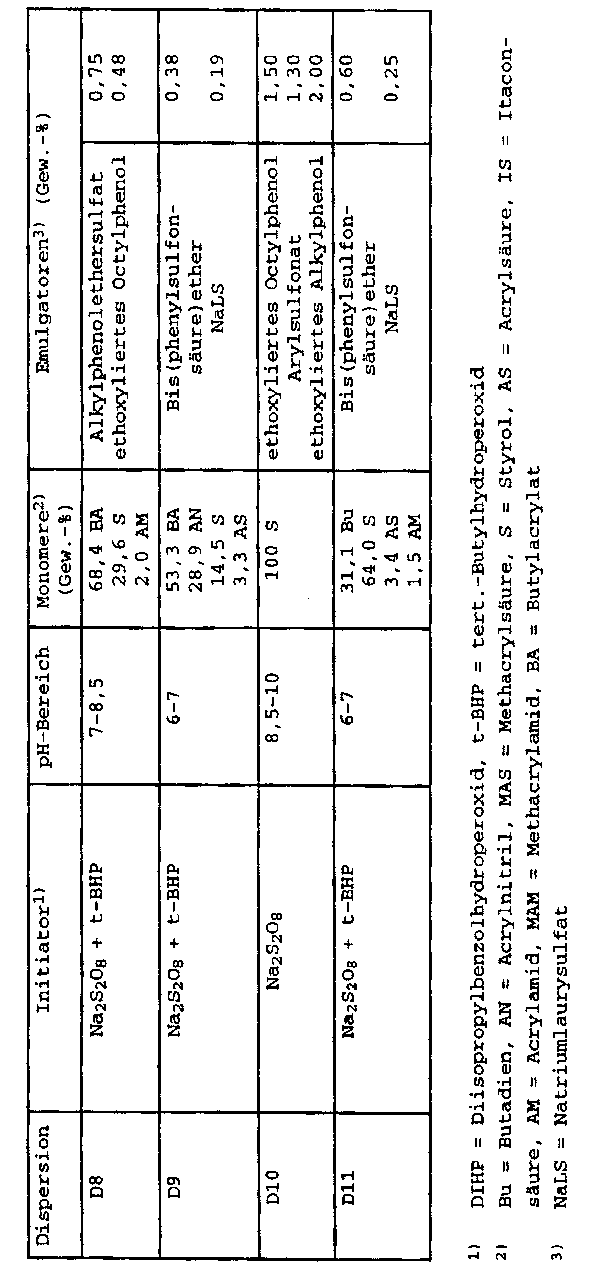

**Tabelle 2**

| Dispersion | Wachstum Easycult-TTC Färbung¹⁾ | Lumineszenz-Schnelltest RLU |
|---|---|---|
| D1 | ++ | 3,5 x 10⁴ |
| D2 | + | 4,0 x 10⁴ |
| D3 | + | 2,1 x 10⁴ |
| D4 | ++ | 5,6 x 10³ |
| D5 | +++ | 4,0 x 10² |
| D6 | + | 7,3 x 10³ |
| D7 | ++ | 9,7 x 10² |
| D8 | + | 8,1 x 10³ |
| D9 | + | 1,6 x 10³ |
| D10 | + | 4,1 x 10² |
| D11 | ++ | 8,1 x 10² |

| | | |
|---|---|---|
| ¹⁾ +++ = sehr starke Rotfärbung, ++ = starke Rotfärbung, + = viele rote Punkte, o = Spur, - = negativ | | |

### Beispiel 2

### Mikrobennachweis in Proben mit bekannter Keimzahl

Aus der Dispersion D6 (Feststoffgehalt etwa 50 Gew.-%) wurden fünf Proben entnommen, die, weil direkt aus der Produktion gezogen, steril waren. Eine Probe (0) wurde als Nullprobe belassen. Allen anderen Proben (D6-1 bis D6-4) wurden jeweils drei Teilproben entnommen, und mit 10³, 10⁶ bzw. 10⁸ Mikroorganismen pro ml Probe aus einer frisch angezüchteten Stammsuspension der Mikroorganismen (Hefen und Bakterien) versetzt. D6-1 wurde mit Hefezellen der Gattung Candida tropicalis, D6-2 und D6-4 wurden mit Gram-negativen Bakterien (E. coli bzw. Burkholderin cepacia) und D6-3 wurde mit Gram-positiven Bakterien (Rhodococcus erythropolis) dotiert. Die Null- und Teilproben wurden mit herkömmlichen Wachstumstests (Agarplattierung bzw. Easycult-TTC) und nach Verdünnung im Verhältnis 1:50 (Gewichtsverhältnis etwa 1 Teil Polymer auf etwa 100 Teile Wasser) mit vollentsalztem sterilem Wasser mit dem Lumineszens-Schnelltest untersucht. Der Lumineszenz-Test wurde wie in Beispiel 1 beschrieben durchgeführt, jedoch unter Verwendung des "Low Decay Rate Bioluminescence Kit" der Fa. Celsis anstelle des "Hygiene Monitoring Kit". Die Dotierungen der D6-Proben wurden durch den Lumineszenz-Test ausnahmslos nachgewiesen, die Zahl der gemessenen RLU lag um etwa eine halbe bis zwei Größenordnungen über der Zahl der für die Nullprobe gemessenen RLU. Die Wachstumstests konnten hingegen einige Kontaminationen nicht nachweisen. Die Meßergebnisse für die einzelnen Proben sind der Tabelle 3 zu entnehmen.

**Tabelle 3**

| Dispersion | Dotierung | | Wachstum | | Lumineszenz-Schnelltest |
|---|---|---|---|---|---|
| | Probe | Zielmenge | Agar-Platten | Easycult-TTC¹⁾ | Verdünnung 1:50 (RLU) |
| | | Keime/ml | Keime/ml | | |
| D6 | 0 | - | - | - | 5,8 x 10² |
| | D6-1 | 10³ | 1 x 10² | - | 1,1 x 10³ |
| | | 10⁶ | 1 x 10⁵ | - | 1,3 x 10³ |
| | | 10⁸ | 1 x 10⁷ | o | 4,9 x 10³ |
| | D6-2 | 10³ | 2 x 10² | o | 1,1 x 10³ |
| | | 10⁶ | 2 x 10⁴ | + | 2,2 x 10³ |
| | | 10⁸ | 4 x 10⁷ | + | 4,6 x 10⁴ |
| | D6-3 | 10³ | 2 x 10¹ | o | 1,5 x 10³ |
| | | 10⁶ | 2 x 10⁴ | + | 2,0 x 10³ |
| | | 10⁸ | 2 x 10⁷ | + | 3,1 x 10⁵ |
| | D6-4 | 10³ | - | o | 1,5 x 10³ |
| | | 10⁶ | - | o | 3,5 x 10³ |
| | | 10⁸ | - | o | 2,8 x 10⁵ |

| | | | | | |
|---|---|---|---|---|---|
| ¹⁾ + = positiv, o = Spur, - = negativ | | | | | |

Der Lumineszens-Schnelltest zum Nachweis von Mikroorganismen funktioniert also überraschenderweise in Polymerdispersionen zuverlässiger als die klassischen Wachstumstests.

## Patentansprüche

1. Verwendung eines Biolumineszenz-Tests zum Nachweis von Mikroorganismen in Dispersionen, die Polymere und/oder Pigmente enthalten.

2. Verwendung nach Anspruch 1, dadurch gekennzeichnet, daß die Dispersionen ausgewählt sind unter Dispersionen auf Vinylchlorid-, Butadien-, Styrol- oder Acrylatbasis.

3. Verwendung nach Anspruch 2, wobei es sich um Reinvinylchlorid-, Reinstyrol-, Reinacrylat-, Reinbutadien-, Styrol-Butadien-, Butadien-Acrylnitril-, Styrol-Butadien-Acrylnitril, Styrol-Acrylnitril oder Acrylat-Styrol-Dispersionen handelt.

4. Verwendung nach einem der vorhergehenden Ansprüche, dadurch gekennzeichnet, daß die Dispersion einen Feststoffgehalt im Bereich von 10 bis 80 Gew.-% besitzt.

5. Verfahren zum Nachweis von Mikroorganismen in Dispersionen, die Polymere und/oder Pigmente enthalten, dadurch gekennzeichnet, daß man
a) eine Probe der Dispersion mit Wasser verdünnt,
b) Mikroorganismen-lysierende Agenzien zu der verdünnten Probe gibt und einwirken läßt,
c) eine Mischung aus Luziferin und Luziferase zugibt und
d) die Lumineszenz der Probe mißt.

6. Verfahren nach Anspruch 5, dadurch gekennzeichnet, daß man die Probe der Dispersion bis zu einem Gewichtsverhältnis von 1 Teil Polymer auf 10 bis 300 Teile Wasser, insbesondere von 1 Teil Polymer auf 100 bis 200 Teile Wasser verdünnt.

7. Verfahren nach Anspruch 5 oder 6, dadurch gekennzeichnet, daß man die Mikroorganismen-lysierenden Agenzien auswählt unter kationischen Tensiden, anionischen Tensiden und nichtionischen Tensiden, insbesondere unter kationischen Tensiden.

8. Verfahren nach Anspruch 7, dadurch gekennzeichnet, daß man als kationisches Tensid ein quaternäres Ammoniumsalz verwendet.

9. Verfahren nach einem der Ansprüche 5 bis 8, dadurch gekennzeichnet, daß man einer verdünnten Probe einer nichtionischen oder anionischen Dispersion ein kationisches Tensid als Mikrorganismen-lysierendes Agens zusetzt.

10. Verfahren nach einem der Ansprüch 5 bis 9, dadurch gekennzeichnet, daß man die Mikroorganismen-lysierenden Agenzien in einer Konzentration von 0,01 bis 5 Gew.-% einsetzt.

11. Verfahren nach einem der Ansprüche 5 bis 10, dadurch gekennzeichnet, daß man als Luziferin Photinus-Luziferin und als Luziferase Photinus-Luziferase verwendet.

12. Verfahren nach einem der Ansprüche 5 bis 11, dadurch gekennzeichnet, daß min die Mikroorganismen-lysierenden Agenzien mindestens 5 bis 20 sec., insbesondere mindestens 10 sec. einwirken läßt.

13. Verfahren nach einem der Ansprüche 5 bis 12, dadurch gekennzeichnet, daß man die Lumineszenz mindestens 10 bis 20 sec., insbesondere mindestens 15 sec. unmittelbar nach Zugabe des Luziferin/Luziferase-Gemisches mißt.
